# EUROPÄISCHE PATENTANMELDUNG

(11) **EP 0 969 001 A2**
(43) Veröffentlichungstag der Anmeldung: **05.01.2000**
(21) Anmeldenummer: 99111617.9
(22) Anmeldetag: 16.06.1999
(51) Int. Cl.: C07C 401/00

(54) **Verfahren zur Herstellung von Vitamin D3 oder Prävitamin D3**

(30) Priorität: 23.06.1998 EP 98111490
(71) Anmelder: F. HOFFMANN-LA ROCHE AG, 4070 Basel (CH)
(72) Erfinder: Johannsen, Monika, 21075 Hamburg (DE)
(74) Vertreter: Buntz, Gerhard

(57) **Zusammenfassung**

Ein Verfahren zum Gewinnen von Vitamin D3 oder Prävitamin D3 aus einem Isomeren-gemisch besteht darin, dass eine Abtrennung mittels Säulenchromatographie mit überkritischen oder flüssigen Kohlendioxid ggf. mit Modifier als mobiler Phase durchgeführt wird, wobei ein ggf. modifiziertes Kieselgel als stationäre Phase verwendet wird.

## Beschreibung

Die vorliegende Erfindung betrifft ein Verfahren zum Gewinnen von Vitamin D₃ oder Prävitamin D₃ aus Gemischen mit anderen Komponenten, z.B. Dehydrocholesterol, Tachysterol und Lumisterol, durch Säulenchromatographie.

Die D-Vitamine sind biologische Wirkstoffe, die für die Regulation des Calcium-Haushaltes bei höheren Tieren unerlässlich sind. Die verschiedenen D- Vitamine unterscheiden sich durch die Natur der Seitenkette. Die praktisch wichtigsten Vertreter sind Vitamin D₂ (Ergocalciferol) und Vitamin D₃ (Cholecalciferol). D-Prävitamine sind bei höheren Tieren und Pflanzen weit verbreitet. Durch UV-Bestrahlung erfolgt eine ausreichende Photo-Aktivierung des Prävitamin D₃. Die zusätzliche Bezeichnung des Vitamin D₃ als antirachitisches Vitamin ist historisch zu verstehen. In unseren Breiten entsteht Rachitis heute in der Regel nicht durch Mangel an Prävitamin, sondern durch Mangel an Sonnenlicht. Die technische Gewinnung der D-Vitamine erfolgt heute durch Umwandlung natürlicher Vorstufen, welche dem Cholesterin nahestehen.

Vitamin D₃ ist in Wasser nicht, in fetten Ölen schwer und in Ethanol, Chloroform, Ether, sowie Aceton gut löslich. Vitamin D₃ ist gegen Licht, Luft, Wärme und Säure empfindlich. Die Schmelztemperatur von Vitamin D₃ liegt im Bereich von 84-87 C. Die Löslichkeit von D-Vitaminen in über- oder unterkritischen Fluiden, z.B. in überkritischem CO₂ im Temperaturbereich von 40 bis 60 C und einem Druckbereich von 20 bis 35 MPa, ist aus der Literatur bekannt. Das technische Verfahren zur Synthese von Vitamin D₃ basiert auf der Bestrahlung von 7-Dehydrocholesterol (DHC), welches aus Cholesterin hergestellt wird. DHC wird durch Bestrahlung in Prävitamin D₃ verwandelt und dieses durch mildes Erwärmen zu Vitamin D₃ isomerisiert. Beim Bestrahlen von DHC entstehen ausserdem Lumisterol und Tachysterol. Die Ausbeute an Prävitamin D₃ und damit an Vitamin D₃ hängt wesentlich von den Bestrahlungsbedingungen ab.

Zur Aufreinigung der Mutterlauge im Anschluss an die Belichtung sind verschiedene Verfahren üblich. So wird z.B. bisher das unerwünschte Tachysterol mittels Diels-Alder-Reaktion in ein Tachysterol-Addukt-di-K-Salz überführt und letzteres anschliessend abgetrennt.

Das herkömmliche Verfahrens weist eine Reihe von Nachteilen auf. Die Ausbeute ist begrenzt durch den Gleichgewichtszustand in der Belichtungsreaktion. Die Durchführung der Diels-Alder-Reaktion benötigt weitere Chemikalien und zeigt keine vollständige Ausbeute von Vitamin D3 oder Prävitamin D3 zur Rohware. Zur Aufreinigung zur kristallinen Qualität sind weitere Reaktionen nötig, bei denen Chemikalien wie Pyridin und Buttersäurechlorid verwendet werden, wobei wiederum keine vollständige Umsetzung erfolgt. Insgesamt ergeben sich also Verluste des Wertproduktes

Der Erfindung liegt die Aufgabe zugrunde, diese Nachteile bei der Gewinnung von Prävitamin oder Vitamin D₃ aus einem Isomerengemisch, wie es z.B. bei der Anwendung des Bestrahlungsverfahren entsteht, zu vermeiden.

Erfindungsgemäss wird dies dadurch erreicht, dass Vitamin bzw. Prävitamin D₃ säulenchromatographisch von dem Gemisch abgetrennt wird.

Vorzugsweise wird dabei als mobile Phase überkritisches oder flüssiges Kohlendioxid unter Zusatz eines polaren Modifiers, z.B. Ethanol, und als stationäre Phase ein ggf. modifiziertes Kieselgel verwendet.

Im folgenden wird anhand der beiliegenden Zeichnungen ein bevorzugtes Ausführungsbeispiel der Erfindung beschrieben. Es zeigen
Fig. 1 ein Fliessdiagramm der einzelnen Verfahrensschritte
Fig.2 ein Blockschema der verwendeten Apparatur

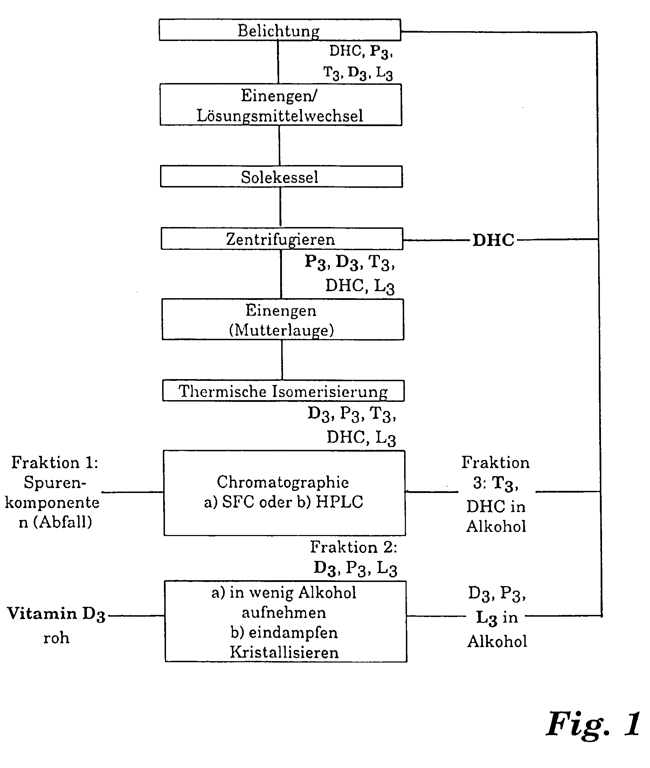

Wie in Fig. 1 dargestellt ist, wird die Mutterlauge zunächst thermisch isomerisiert, dann chromatographiert und dabei das restliche 7-Dehydrocholesterol (DHC) sowie das Tachysterol (T₃) entfernt und in den Belichtungsansatz zurückgeführt. Da es sich bei der photochemischen Reaktion um eine Gleichgewichtsreaktion handelt, verhindert das Zuführen der eigentlich unerwünschten Komponenten das erneute Entstehen derselben, so dass sich eine Erhöhung der Ausbeute ergibt. Aus der gewonnenen Wertfraktion (Fraktion 2) kann Vitamin D₃ kristallisiert werden. Der in Lösung verbleibende Anteil der Substanzen D₃, Prävitamin D₃ (P₃) und Lumisterol (L₃) wird ebenfalls in den Belichtungsansatz zurückgeführt. Falls gewünscht kann Fraktion 2 auch weiter chromatographisch aufgetrennt werden.

Es ergeben sich folgende Vorteile eines chromatographischen Verfahrens:
- Vermeidung der Diels-Alder-Reaktion
- Nebenfraktionen werden in den Prozess zurückgeführt
- höhere Ausbeute
- reineres Produkt;
   und speziell bei Einsatz von SFC (Chromatographie mit überkritischen Gasen):
- lösungsmittelfreier Prozessschritt
- einfache Abtrennung durch Entspannung
- Eluent ist problemlos im Kreis zu führen.

Im Prinzip wird das erfindungsgemässe Verfahren so durchgeführt, dass man das gewünsehtenfalls bereits unter Druck stehende Isomerengemisch mit der überkritischen oder flüssigen mobilen Phase zusammenbringt, das Ganze eventuell gefolgt von weiterer mobiler Phase, auf die mit der oben erwähnten stationären Phase gepackte Chromatographiesäule gibt und dann durchströmen (eluieren) lässt, wobei das Eluieren unter den gewählten Temperatur- und Druckbedingungen erfolgt und aufgrund der starken Wechselwirkungen zwischen stationärer Phase und den einzelnen Bestandteilen des Gemisches eine zeitliche Auftrennung dieser Bestandteile erreicht wird, die aus der Säule nacheinander eluierenden, in mobiler Phase gelösten Bestandteile (Eluate) nach sequentieller Detektion (Bestimmung) in durch das Detektionsmittel bestimmten Auffanggefässen sammelt und das Kohlendioxid von jeweiligen Sammelgut durch Dekomprimieren (Verflüchtigung) entfernt, so dass sich schliesslich die erhaltenen, abgetrennten Bestandteile oder "Fraktionen" (u.a. das erwünschte Vitamin D₃ oder Prävitamin D₃) kohlendioxidfrei in den einzelnen Auffanggefässen befinden. Gewünschtenfalls kann nach dem Eluieren und Austritt aus der Säule das Eluat einem oder mehreren weiteren derartigen chromatographischen Vorgängen unterworfen werden, um eine noch grössere Aufteilung der Bestandteile zu erzielen. Dies gilt auch für eine beliebige Fraktion, falls deren Reinheit der angestrebten nicht entspricht.

Als im erfindungsgemässen Verfahren verwendetes Gemisch von Vitamin D₃-Isomeren eignet sich jedes beliebiges Gemisch, das Vitamin D₃ oder Prävitamin D₃ enthält. So kann man beispielsweise ein synthetisches Gemisch vor oder nach der thermischen Isomerisierung verwenden.

Das Isomerengemisch mit Vitamin D₃ und/oder Prävitamin D₃ wird normalerweise ohne Verdünnung zusammen mit der überkritischen oder flüssigen mobilen Phase auf die mit der erfindungsgemäss verwendeten stationären Phase gepackte Chromatographiesäule gegeben, kann allerdings im voraus in einem geeigneten Lösungsmittel, z.B. einem niederen Alkanol, vorzugsweise Ethanol, gelöst werden. Vorzugsweise wird das Gemisch jedoch unverdünnt eingesetzt.

Bei dem im erfindungsgemässen Verfahren verwendeten überkritischen Kohlendioxid handelt es sich bekanntlich um die Form von Kohlendioxid, die bei einer Temperatur von mindestens etwa 31°C und bei einem Druck von mindestens etwa 7.3 MPa gehalten wird und weder rein flüssig noch rein gasförmig, sondern ein Hybrid der beiden physikalischen Formen ist. Das im erfindungsgemässen Verfahren als Alternative verwendete flüssige Kohlendioxid weist eine Temperatur von weniger als etwa 31°C und einen Druck, der oberhalb von etwa 7.3 MPa liegt, auf Die Vorteile der Verwendung von Kohlendioxid bestehen in dessen Nichttoxizität, Nichtflammbarkeit und leichter Entfernung durch Dekomprimieren der aufgefangenen Eluate, ohne dass ein potentiell schädigender Rückstand mit der abgetrennten Fraktionen (z.B. Vitamin D₃ oder Prävitamin D₃) davon bleibt. Ferner ist das Kohlendioxid in hoher Reinheit und kostengünstig breit erhältlich und lässt sich gewünschtenfalls mit einem organischen Cosolvens (Modifier), z.B. dem bereits erwähnten Ethanol, aber auch mit anderen Alkanolen (z.B. Methanol), Alkanen (z.B. n-Hexan) oder Ketonen (z.B. Aceton) als Teil der mobilen Phase verwenden. Da die kritische Temperatur von Kohlendioxid nicht viel höher ist als die Raumtemperatur und die erfindungsgemäss zu gewinnenden Substanzen temperaturempfindlich (thermolabil) sind, eignet sich Kohlendioxid auch aus diesen Gründen hervorragend als im erfindungsgemässen Verfahren verwendete mobile Phase.

Das im erfindungsgemässen Verfahren als stationäre Phase verwendete modifizierte Silicagel liegt zweckmässigerweise als möglichst homogene gepackte ungleichmässige oder, vorzugsweise, kugelförmige Partikel vor, deren Partikelgrösse etwa 5 bis 25 mm beträgt. Als Beispiele kommerziell erhältlicher Silicagele seien Zorbax und Hyperprep erwähnt. Das erste weist eine spezifische Oberfläche von S_{BET} von 350 m²/g, ein Porenvolumen Vₚ von 0.53 ml/g, einen Porendurchmesser D von 60 oder 150 Å und eine Partikelgrösse dp₅₀ von 10 mm auf, während das letztere eine S_{BET} von 300 m²/g, ein Vₚ von 0.7 ml/g, einen D von 100 Å und eine Partikelgrösse dp₅₀ von 12 mm aufweist.

Damit das als mobile Phase im erfindungsgemässen Verfahren verwendete Kohlendioxid im überkritischen oder flüssigen Bereich beibehalten wird, müssen gewisse Temperatur- und Druckbedingungen eingehalten werden, und zwar nicht nur bei der Einführung des Kohlendioxids in die mit stationären Phase gepackte Chromatographiesäule, sondern auch noch während des anschliessenden Eluierens. Das Verfahren wir zweckmässigerweise im Temperaturbereich von 0°C bis etwas 100°C und bei einem Druck von etwa 7.5 MPa bis etwa 32.0 MPa durchgeführt. Vorzugsweise beträgt der Temperaturbereich etwa 30°C bis etwa 60°C und der diesbezügliche Druckbereich 7.5 bis 15.0 MPa. Die Dichte von Kohlendioxid lässt sich über Druck und Temperatur einstellen, und in dem letzterwähnten Temperatur- und Druckbereich ergibt sich eine Dichte von etwa 170 kg/m³ bis etwa 850 kg/m³.

Sowohl die Temperatur- und die Druckbedingungen, unter denen das erfindungsgemässe Verfahren durchgeführt wird, als auch die Wahl der stationären Phase und der mobilen Phase, üben einen Einfluss auf das Trennergebnis aus. Im allgemeinen zieht eine Temperaturerhöhung oder Druckerniedrigung die Eluate der verschiedenen Isomeren zeitlich auseinander, während eine Druckerhöhung oder Temperaturerniedrigung die Eluate zusammenschiebt, so dass das beliebige Variieren dieser Parameter den zeitlichen Ablauf des erfindungsgemässen Verfahrens bestimmen kann.

### ggf. Einfluss verschiedener Mischungen der mobilen Phase

Die Detektion der auf der Chromatographiesäule nacheinander eluierenden, in Kohlendioxid gelösten Bestandteile (Eluate) erfolgt parallel vorzugsweise über einen UV-Detektor und einen Flammenionisationsdetektor (FID). Unter Verwendung der Detektion wird die Zuteilung der verschiedenen Eluate in die Auffanggefässe elektronisch besorgt. Derartige Technologie ist an sich bekannt, wie auch die Art und Weise, durch die das Kohlendioxid (durch Dekomprimieren) vom jeweiligen Sammelgut entfernt wird.

Die Erfindung wird anhand des nachfolgenden Beispiels veranschaulicht.

### Beispiel

Für eine Untersuchung des chromatographischen Gewinnens der Bestandteile, insbesondere von Vitamin D₃ oder Prävitamin D₃ aus einem Isomerengemisch wird eine Apparatur der Firma Hewlett Packard (HP G1205A SFC) verwendet. Die Apparatur besteht aus den Grundbausteinen Pumpe, Ofen mit Gasphasendetektor, externer Detektor und Autosampler. In Abb. 3 ist ein Fliessschema der Apparatur zu sehen. Die Apparatur wird kontinuierlich mit flüssigem Kohlendioxid versorgt. Die mobile Phase kann je nach gewählten Druck- und Temperaturbedingungen im überkritischen (bei reinem CO₂ etwa über 31°C und 7.3 MPa) oder auch unterkritischen Bereich betrieben werden.

Die Apparatur wurde im sogenannten Downstream-Modus betrieben. Diese Betriebsweise bedeutet, dass bei einem Einsatz von gepackten Säulen mit einem Innendurchmesser grösser 1 mm neben dem Fluss, der Säulenhinterdruck vom System als feste Regelgrösse genutzt wird. Die Speisung der Pumpe erfolgt durch das hausinterne Gasnetz mit flüssigem Hochdruck-CO₂ (P » 35 MPa). Mittels eines Druckminderers wird der Eingangsdruck der Pumpe auf einen Druck von Pₑᵢₙ » 10 MPa reduziert. Diese Einstellung kann in gewissen Grenzen variiert werden, dabei sollte sichergestellt sein, dass die Pumpe mit Flüssigphase gespeist wird. Die Förderung und Verdichtung aufden nötigen Säulenvordruck erfolgt mittels einer Kolbenpumpe. Der Pumpenkopf hat eine Temperatur von 278 K, um die entstehende Kompressionswärme abzuführen. Die analytische Säule befindet sich im Ofen, in dem auch die mobile Phase auf die Versuchstemperatur erhitzt wird. Die Probenaufgabe erfolgt durch ein pneumatisch gesteuertes 4-Wege-Rheodyne-Ventil, das mit einer internen Schleife von 5 ml ausgestattet ist. Der automatische Probengeber, der mit einer 50 ml Spritze ausgestattet ist, füllt die interne Schleife über den Injektionsport mit Probenlösung. Die Probe gelangt dann mit der mobilen Phase auf die Säule. Hier findet aufgrund unterschiedlich starker Wechselwirkungen zwischen der stationären Phase und den einzelnen Bestandteilen der Probelösung eine Auftrennung des Gemisches statt. Die Bestandteile des Gemischen werden (im Idealfall) nacheinander von der Säule eluiert. Hinter der analytischen Säule erfährt der Eluentenstrom einen permanenten Split. Dieser Split wird durch einen fixen Restriktors erzielt, der den Splitstrom zum Gasphasendetektor, einem Flammenionisationsdetektor (FID), führt. Der nach dem Split verbleibende Grossteil des Eluentenstrom passiert den Diodenarraydetektor (DAD). Dem DAD nachgeschaltet ist die Entspannungseinheit des SFC.

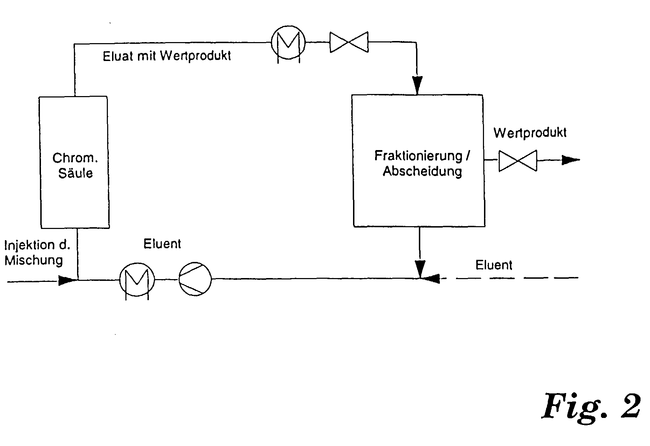

Die Chromatogramme werden mit dem Datensystem aufgezeichnet. Die Untersuchungen mittels analytischer SFC zeigen eine erfolgreiche Trennung der Vitamin D₃-Isomeren. Die Trennung mit CO₂ als Eluent ohne Modifier ist in diesem Fall nicht möglich. Ausgesprochen gute Ergebnisse hingegen werden durch den Zusatz geringer Mengen Alkohol zum CO₂ erzielt. Als stationäre Phase werden verschiedene Normalphasenmaterialien auf Silicabasis eingesetzt. Die Selektivität zwischen Vitamin D₃ und Tachysterol liegt z.B. auf einer Cyanophase bei α » 1,5 (siehe Figur 2), was für eine präparative Trennung optimal ist. Die Art des Alkohols (Methanol, Ethanol, iso-Propanol) zeigt nahezu keinen Einfluss. Die Selektivität erhöht sich bei gleichzeitiger Retentionszeitverlängerung je geringer der Modifieranteil ist. Die Retentionszeit lässt sich in gewissem Rahmen durch Steigerung der Dichte des CO₂ (bzw. der mobilen Phase) verkürzen.

## Patentansprüche

1. Verfahren zum Gewinnen von Vitamin D₃ oder Prävitamin D₃ aus Gemischen mit anderen Komponenten, z.B. Dehydrocholesterol, Lumisterol und Tachysterol, gekennzeichnet durch Abtrennung des Vitamin D₃ oder Prävitamin D₃ mittels Säulenchromatographie.

2. Verfahren nach Anspruch 1, dadurch gekennzeichnet, dass als mobile Phase überkritisches oder flüssiges Kohlendioxid und Modifier verwendet wird.

3. Verfahren nach Anspruch 1, dadurch gekennzeichnet, dass als stationäre Phase ein ggf. modifiziertes Kieselgel verwendet wird.

4. Verfahren nach Anspruch 1, dadurch gekennzeichnet, dass das ggf. modifizierte Kieselgel als homogen gepackte, kugelförmige Partikel vorliegt, deren Partikelgrösse etwa 5 bis 25 mm beträgt.

5. Verfahren nach einem der Ansprüche 1 - 4, dadurch gekennzeichnet, dass als Gemisch von Vitamin D₃-Isomeren ein Reaktionsgemisch aus der synthetischen Herstellung nach dem Bestrahlungsverfahren verwendet wird.

6. Verfahren nach einem der Ansprüche 1 bis 5, dadurch gekennzeichnet, dass seine Durchführung im Temperaturbereich von etwa 30°C bis etwa 60°C nd im Druckbereich etwa 7.0 bis etwa 15.0 MPa erfolgt.
